# EUROPEAN PATENT APPLICATION

(11) **EP 4 260 898 A1**
(43) Date of publication of application: **18.10.2023**
(21) Application number: 21917564.3
(22) Date of filing: 29.10.2021
(51) Int. Cl.: A61M 39/10, A61M 39/26

(54) **MALE CONNECTOR AND MEDICAL APPLIANCE**

(30) Priority: 08.01.2021 JP 2021002397
(71) Applicant: TERUMO Kabushiki Kaisha, Tokyo 151-0072 (JP)
(72) Inventor: KURIYAMA Tasuku, Nakakoma-gun, Yamanashi 409-3853 (JP)
(74) Representative: Casalonga
(86) International application number: PCT/JP2021/040134
(87) International publication number: WO 2022/149339

(57) **Abstract**

A male connector according to the disclosure is a male connector connectable to a female connector provided with an elastic valve body, and the male connector includes a housing, a flow passage tubular member having an opening formed thereon, and a valve body. The housing includes a housing body, and a movable body that deforms or moves the valve body so as to change a configuration between a first configuration in which the opening of the flow passage tubular member is closed by the valve body, and a second configuration in which the opening of the flow passage tubular member is freed from the valve body. The movable body includes a first locking portion that is capable of locking the female connector in the first configuration, and the housing body includes a second locking portion that is capable of locking the female connector in the second configuration.

## Description

### Technical Field

The present disclosure relates to a male connector and a medical device.

### Background Art

When an infusion into a patient is performed, it is necessary to form a path (an infusion line) for transporting a fluid such as medical fluids. The infusion line is generally formed by connecting infusion tubes or the like. When a fluid such as medical fluids to be administered to a patient is injected into a medical fluid bag, it is necessary to connect the medical fluid bag and a syringe or the like.
In order to detachably interconnect different members in this way, a male connector and a female connector are used.

Among the medical fluids, for example, there is a medical fluid containing a drug designated as a dangerous drug such as an anti-cancer agent, and a medical staff needs to pay careful attention, for example, at the time of attaching and detaching the male connector and the female connector to prevent a fluid such as the dangerous medical fluid from adhering to a finger or the like.

### Citation List

### Patent Literature

PTL 1: WO 2018/056465

### Summary of Invention

### Technical Problem

PTL 1 discloses a male connector that has a configuration capable of restraining leakage of a fluid such as medical fluids to the outside when connection with a female connector is released. According to the male connector described in PTL 1, it is possible to restrain a liquid such as medical fluids from adhering to a finger of a medical staff.

The male connector described in PTL 1 has room for further improvement from the viewpoint of connection stability with the female connector.

An object of the disclosure is to provide a male connector that is capable of improving connection stability with a female connector and restraining leakage of a fluid such as medical fluids to the outside when connection with the female connector is released, and a medical device provided with the male connector.

### Solution to Problem

A male connector according to a first aspect of the disclosure is a male connector connectable to a female connector provided with an elastic valve body, and the male connector includes: a housing configured to define a hollow portion; a flow passage tubular member extending in the hollow portion and having an opening formed at one end side in an extending direction; and a valve body positioned in the hollow portion and configured to close the opening of the flow passage tubular member. The housing includes a housing body, and a movable body configured to move in the extending direction with respect to the housing body to deform or move the valve body such that a configuration is changed between a first configuration in which the opening of the flow passage tubular member is closed by the valve body, and a second configuration in which the opening of the flow passage tubular member is freed from the valve body. The movable body includes a first locking portion configured to lock the female connector in a state where the valve body is in contact with the elastic valve body of the female connector in the first configuration, and, the housing body includes a second locking portion configured to lock the female connector in a state where the first locking portion locks the female connector in the second configuration.

As an embodiment of the disclosure, the movable body is provided with only one first locking portion or a plurality of the first locking portions at intervals in a circumferential direction of the flow passage tubular member.

As an embodiment of the disclosure, when the movable body moves in the extending direction with respect to the housing body, the first locking portion of the movable body is movable in a radial direction of the flow passage tubular member.

As an embodiment of the disclosure, the housing body is provided with only one second locking portion or a plurality of the second locking portions at intervals in the circumferential direction of the flow passage tubular member.

As an embodiment of the disclosure, when a state where the second locking portion of the housing body locks the female connector is released, the movable body moves in the extending direction with respect to the housing body due to a restoring force of the deformed valve body or a biasing force of a biasing member that biases the valve body or the movable body toward the one end side in the extending direction, and the configuration is changed from the second configuration to the first configuration.

As an embodiment of the disclosure, after the configuration is changed from the second configuration to the first configuration, the movable body continuously moves in the extending direction with respect to the housing body due to the restoring force or the biasing force, so that the state where the first locking portion locks the female connector is released.

As an embodiment of the disclosure, the movable body includes a first movable body and a second movable body that are relatively movable in the extending direction, and in a state where the first movable body is locked such that the first movable body does not move in the extending direction with respect to the housing body, the second movable body is movable in the extending direction with respect to the first movable body and the housing body.

A medical device as a second aspect of the disclosure includes the male connector.

### Advantageous Effect of Invention

According to the disclosure, it is possible to provide a male connector that is capable of improving connection stability with a female connector and restraining leakage of a fluid such as medical fluids to the outside when connection with the female connector is released, and a medical device provided with the male connector.

### Brief Description of Drawings

[FIG. 1] FIG. 1 is a perspective view illustrating a male connector according to an embodiment of the disclosure, and a female connector connectable to the male connector.
[FIG. 2] FIG. 2 is a side view of the male connector and the female connector illustrated in FIG. 1 before connection.
[FIG. 3] FIG. 3 is a side view of the male connector and the female connector illustrated in FIG. 1 before connection when seen from a position different from that of FIG. 2.
[FIG. 4] FIG. 4 is a cross-sectional view taken along a line I-I in FIG. 2.
[FIG. 5] FIG. 5 is a cross-sectional view taken along a line II-II in FIG. 3.
[FIG. 6] FIG. 6 is a perspective view illustrating a housing body alone of the male connector illustrated in FIG. 1.
[FIG. 7] FIG. 7 is a perspective view illustrating a first movable body alone of the male connector illustrated in FIG. 1.
[FIG. 8] FIG. 8 is an exploded perspective view of a flow passage tubular member, a valve body, and a second movable body of the male connector illustrated in FIG. 1.
[FIG. 9] FIG. 9 is a cross-sectional view at the same position as in FIG. 4 that illustrates a state where the male connector is in progress of connection to the female connector illustrated in FIG. 1.
[FIG. 10] FIG. 10 is a cross-sectional view at the same position as in FIG. 5 of the male connector and the female connector illustrated in FIG. 9 that are in progress of connection to each other.
[FIG. 11] FIG. 11 is a cross-sectional view at the same position as in FIGS. 4 and 9 that illustrates a first connection state of the male connector and the female connector illustrated in FIG. 1.
[FIG. 12] FIG. 12 is a cross-sectional view at the same position as in FIGS. 5 and 10 of the male connector and the female connector in the first connection state illustrated in FIG. 11.
[FIG. 13] FIG. 13 is a cross-sectional view at the same position as in FIGS. 4, 9 and 11 that illustrates a second connection state of the male connector and the female connector illustrated in FIG. 1.
[FIG. 14] FIG. 14 is a cross-sectional view at the same position as in FIGS. 5, 10 and 12 of the male connector and the female connector in the second connection state illustrated in FIG. 13.
[FIG. 15] FIG. 15 is a view illustrating a movement of the second movable body with respect to the first movable body of the male connector illustrated in FIG. 1.
[FIG. 16] FIG. 16 is a diagram illustrating a medical device as an embodiment according to the disclosure.
[FIG. 17] FIG. 17 is a view illustrating a state where a female connector different from the female connector illustrated in FIG. 1 is connected to the male connector illustrated in FIG. 1.

### Description of Embodiments

Hereinafter, embodiments of a male connector and a medical device according to the disclosure will be described with reference to the drawings. In the drawings, common components are denoted by the same reference numerals.

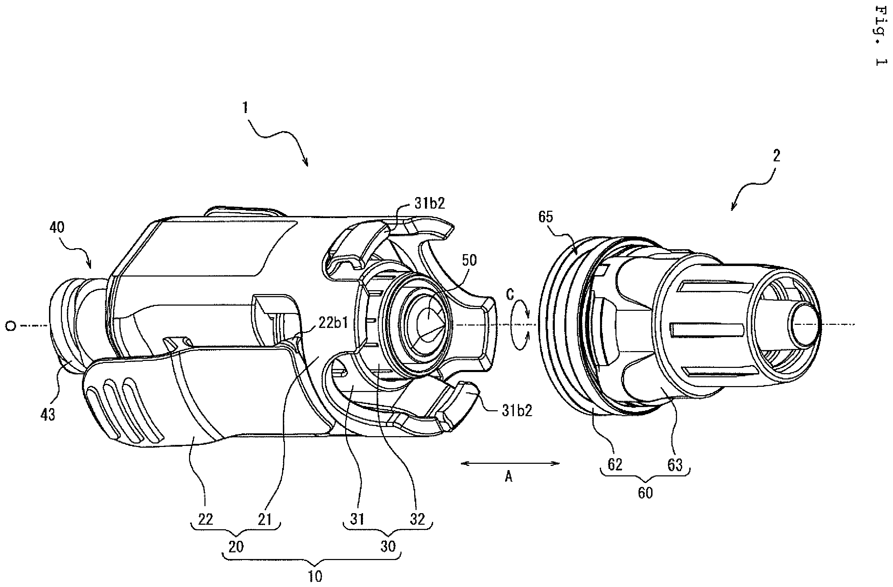

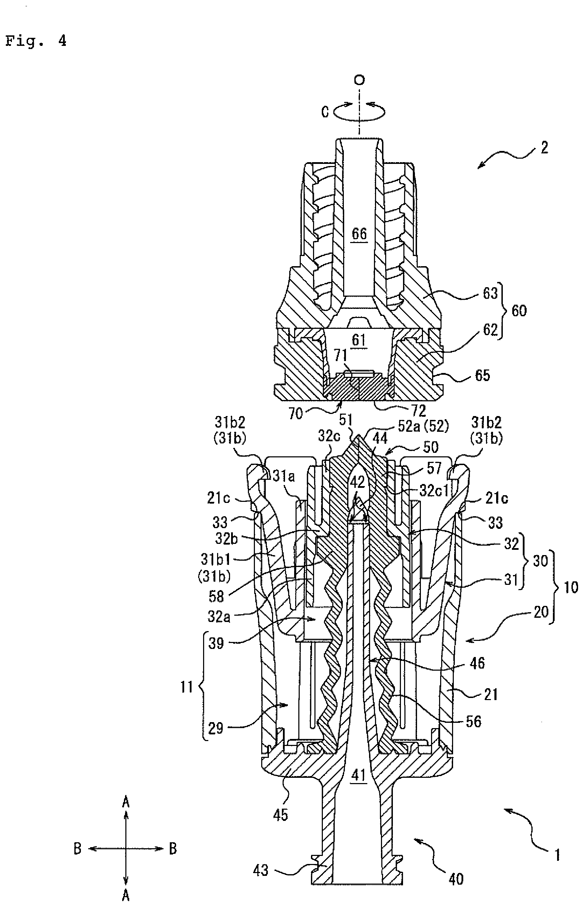

FIG. 1 is a perspective view illustrating a male connector 1 as an embodiment of the male connector according to the disclosure, and a female connector 2 connectable to the male connector 1. The male connector 1 according to the present embodiment is a so-called "closed male connector" in which a flow passage tubular body 46 as a male luer to be described later (see FIG. 4 and the like) is not exposed to the outside in a state where the male connector 1 is not connected to the female connector 2.

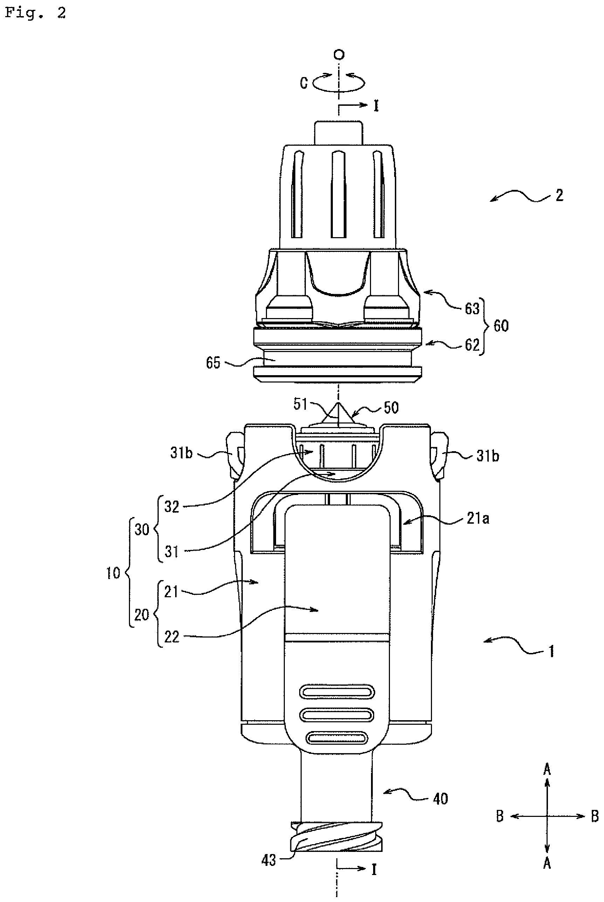

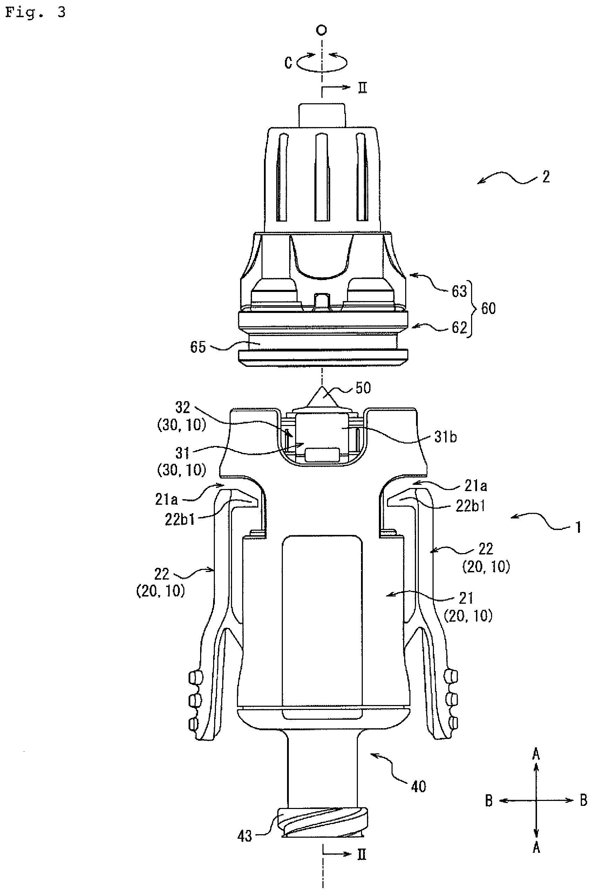

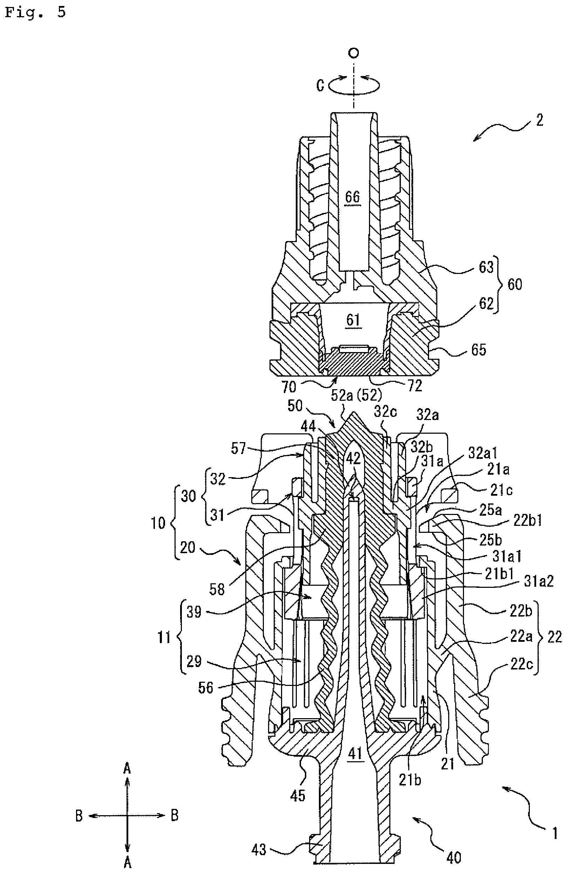

FIGS. 2 and 3 are side views of the male connector 1 and the female connector 2 illustrated in FIG. 1. FIGS. 2 and 3 are side views seen from different positions. FIG. 4 is a cross-sectional view taken along a line I-I in FIG. 2. FIG. 5 is a cross-sectional view taken along a line II-II in FIG. 3. In FIGS. 1 to 5, the male connector 1 and the female connector 2 are illustrated separately in a state before being connected to each other (hereinafter, simply referred to as a "state before connection"). Although details will be described later, the male connector 1 and the female connector 2 according to the present embodiment are connectable simply by an operation of bringing the male connector 1 and the female connector 2 close to each other in an extending direction A of a flow passage tubular member 40 from the state illustrated in FIG. 1. Hereinafter, in the male connector 1 and the female connector 2 according to the present embodiment, one end side, which is a flow passage downstream side (a right direction in FIG. 1, and an upward direction in FIGS. 2 to 5) of a fluid such as medical fluids in an infusion line, is referred to as a "distal side", and the other end side, which is a flow passage upstream side (a left direction in FIG. 1, a downward direction in FIGS. 2 to 5) of the fluid in the infusion line, is referred to as a "proximal side".

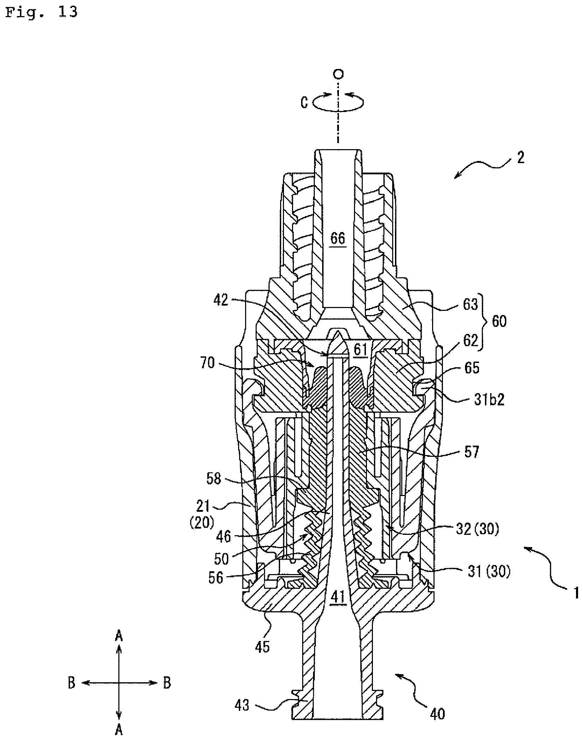

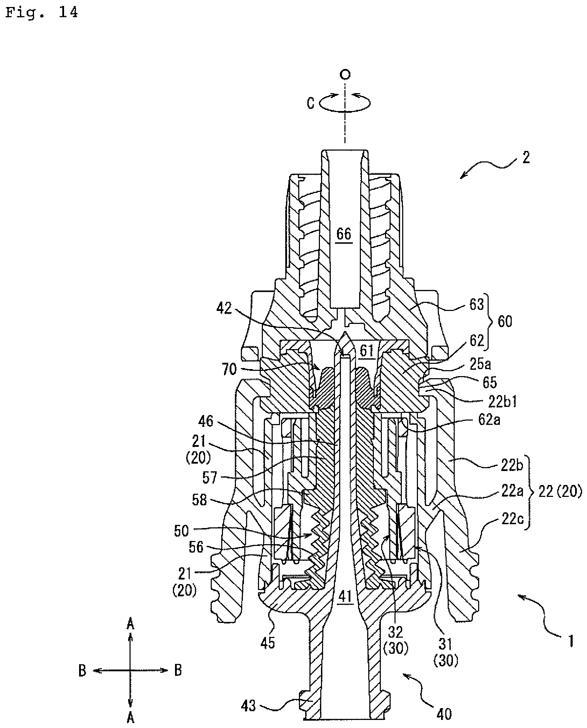

As illustrated in FIGS. 1 to 5, the male connector 1 includes a housing 10, the flow passage tubular member 40, and a valve body 50. As illustrated in FIGS. 4 and 5, the housing 10 defines a hollow portion 11. The flow passage tubular member 40 extends into the hollow portion 11 of the housing 10. Further, an opening 42 is formed at a distal side in the extending direction A of the flow passage tubular member 40. As illustrated in FIGS. 4 and 5, the valve body 50 is positioned in the hollow portion 11 of the housing 10. As illustrated in FIG. 4, the valve body 50 closes the opening 42 of the flow passage tubular member 40. Although details will be described later, configuration of the male connector 1 can be changed between a first configuration in which the valve body 50 closes the opening 42 of the flow passage tubular member 40 (see FIG. 4 and the like), and a second configuration in which the valve body 50 frees the opening 42 of the flow passage tubular member 40 (see FIGS. 13 and 14).

Hereinafter, the extending direction A of the flow passage tubular member 40 will be simply referred to as the "extending direction A". Further, a radial direction B of a virtual circle about a central axis O of the flow passage tubular member 40 is simply referred to as the "radial direction B". In addition, a circumferential direction C around the central axis O of the flow passage tubular member 40 is simply referred to as the "circumferential direction C".

As illustrated in FIGS. 1 to 5, the female connector 2 includes a housing 60 and an elastic valve body 70. As illustrated in FIGS. 4 and 5, the housing 60 defines a male connector insertion portion 61 and a flow passage 66 as hollow portions. The elastic valve body 70 is positioned at a proximal end of the male connector insertion portion 61 of the housing 60, and occludes the male connector insertion portion 61. A top surface 72 of the elastic valve body 70 is exposed to the outside of the female connector 2. The flow passage tubular body 46 (see FIG. 4 and the like) of the flow passage tubular member 40 of the male connector 1, which will be described later, is inserted into the male connector insertion portion 61 from a top surface 72 side of the elastic valve body 70.

Hereinafter, a configuration of the male connector 1 and a configuration of the female connector 2 will be described in detail. Hereinafter, for convenience of description, the housing 10 of the male connector 1 is referred to as a "first housing 10", and the housing 60 of the female connector 2 is referred to as a "second housing 60" . Further, for convenience of description, a flow passage 41 of the male connector 1 is referred to as a "first flow passage 41", and the flow passage 66 of the female connector 2 will be referred to as a "second flow passage 66".

### <Male Connector 1>

### [First Housing 10]

The first housing 10 includes a housing body 20 and a movable body 30 movable with respect to the housing body 20. The hollow portion 11 of the first housing 10 includes a hollow portion 29 defined by the housing body 20, and a hollow portion 39 defined by the movable body 30.

### [[Housing Body 20]]

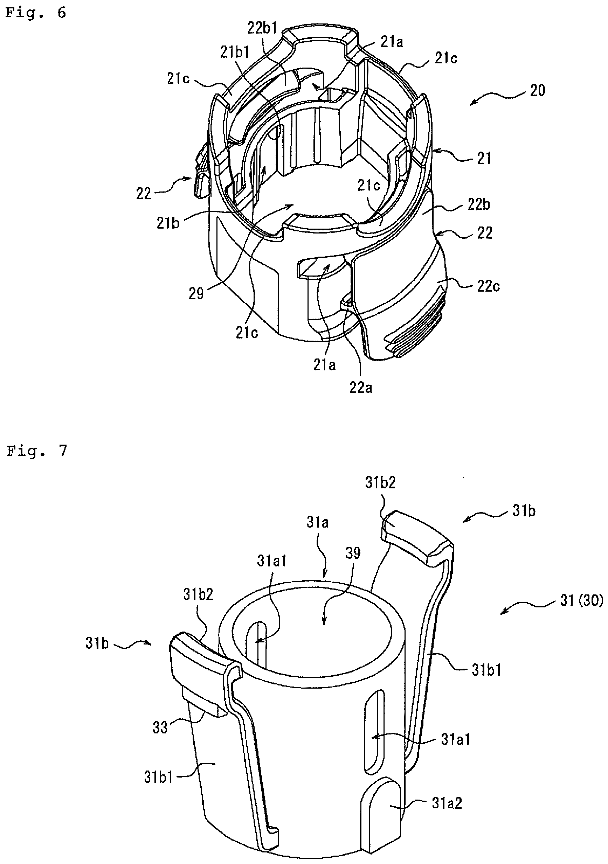

FIG. 6 is a perspective view illustrating the housing body 20 alone. The housing body 20 includes a tubular portion 21 extending in the extending direction A, and locking claw portions 22 protruding from the tubular portion 21. The tubular portion 21 defines openings 21a penetrating in the radial direction B at positions facing each other in the radial direction B. In addition, a plurality of recessed portions 21c recessed toward the proximal side are formed on an end surface of the tubular portion 21 on the distal side in the extending direction A. In the present embodiment, four recessed portions 21c disposed to be separated from one another in the circumferential direction C are formed on the end surface of the tubular portion 21.

As illustrated in FIG. 6, long groove portions 21b extending along the extending direction A are formed on an inner surface of the tubular portion 21. proximal ends of the long groove portions 21b extend to a proximal end of the tubular portion 21, and are opened at the proximal end of the tubular portion 21. On the other hand, distal ends of the long groove portions 21b are terminated at positions of wall portions 21b1 formed in the inner surface of the tubular portion 21.

Each of the locking claw portions 22 includes a support portion 22a, a claw body portion 22b, and an operation portion 22c.

The support portion 22a of the locking claw portion 22 protrudes toward an outer side in the radial direction B from the tubular portion 21. The support portion 22a according to the present embodiment supports the claw body portion 22b and the operation portion 22c. The support portions 22a according to the present embodiment are provided at positions facing each other in the radial direction B of the tubular portion 21. Specifically, the two support portions 22a according to the present embodiment and the two openings 21a of the tubular portion 21 are disposed at the same positions in the circumferential direction C.

The claw body portion 22b protrudes from the support portion 22a toward the distal side in the extending direction A. Each of the claw body portions 22b includes a locking claw 22b1 that protrudes toward an inner side in the radial direction B at a distal end portion which is a tip portion of the claw body portion 22b. The claw body portion 22b can swing in the radial direction B with the support portion 22a as a fulcrum. As the claw body portion 22b swings in the radial direction B, the locking claw 22b1 also moves in the radial direction B. In the state before connection illustrated in FIG. 1 and the like, the locking claw 22b1 of the claw body portion 22b according to the present embodiment enters the opening 21a of the tubular portion 21.

An end surface of the locking claw 22b1 on the distal side includes an inclined surface 25a that is inclined toward the proximal side in the extending direction A from the outer side toward the inner side in the radial direction B. The inclined surface 25a according to the present embodiment extends to an inner end of the locking claw 22b1 in the radial direction B.

An end surface of the locking claw 22b1 on the proximal side includes a hook surface 25b substantially parallel to the radial direction B. The hook surface 25b according to the present embodiment extends to the inner end of the locking claw 22b1 in the radial direction B.

The operation portion 22c protrudes from the support portion 22a toward the proximal side in the extending direction A. The operation portion 22c can swing in the radial direction B with the support portion 22a as the fulcrum. By moving the operation portion 22c toward the inner side in the radial direction B, the claw body portion 22b moves toward the outer side in the radial direction B with the support portion 22a as the fulcrum. That is, by swinging the operation portion 22c in the radial direction B, the claw body portion 22b can be moved in the radial direction B.

### [[Movable Body 30]]

The movable body 30 is movable in the extending direction A with respect to the housing body 20. Since the movable body 30 moves in the extending direction A with respect to the housing body 20, the valve body can be deformed or moved (deformed in the present embodiment) so as to change the configuration between the first configuration and the second configuration. The "first configuration" is a configuration in which the opening 42 of the flow passage tubular member 40 is closed by the valve body 50. As illustrated in FIGS. 4 and 5, in the present embodiment, the valve body 50 covers an end portion of the flow passage tubular member 40 on the distal side in the extending direction A, and closes the opening 42, thereby implementing the first configuration. The "second configuration" is a configuration in which the opening 42 of the flow passage tubular member 40 is freed from the valve body 50. Although details will be described later, in the present embodiment, the flow passage tubular member 40 penetrates the valve body 50, and the opening 42 is exposed to the distal side from the valve body 50, thereby implementing the second configuration (see FIGS. 13 and 14).

The movable body 30 according to the present embodiment includes a first movable body 31 and a second movable body 32. The first movable body 31 and the second movable body 32 are relatively movable in the extending direction A.

FIG. 7 is a perspective view illustrating the first movable body 31 alone. The first movable body 31 includes a tubular body portion 31a and locking claw portions 31b. The tubular body portion 31a has a substantially cylindrical outer shape. As illustrated in FIG. 7, the body portion 31a defines long holes 31a1 penetrating in the radial direction B at positions facing each other in the radial direction B. The long holes 31a1 are openings that have a shape elongated in the extending direction A.

In addition, the body portion 31a includes protrusion portions 31a2 protruding toward the outer side in the radial direction B at positions facing each other in the radial direction B. As illustrated in FIG. 5, the protrusion portions 31a2 are fitted into the long groove portions 21b (see FIG. 6) of the housing body 20 described above. When the protrusion portions 31a2 move along the long groove portions 21b, the first movable body 31 can move in the extending direction A with respect to the housing body 20. The first movable body 31 is movable on the distal side in the extending direction A with respect to the housing body 20 until the protrusion portions 31a2 come into contact with the wall portions 21b1 (see FIG. 6) of the long groove portions 21b. In other words, the long groove portions 21b of the housing body 20 are guide grooves that guide a movement of the first movable body 31 in the extending direction A. Further, the wall portions 21b1 of the long groove portions 21b of the housing body 20 are movement restricting walls that come into contact with the first movable body 31 to restrict the first movable body 31 from further moving toward the distal side in the extending direction A.

The locking claw portions 31b protrude toward the outer side in the radial direction B from the body portion 31a. More specifically, each of the locking claw portions 31b according to the present embodiment includes an arm portion 31b1 that protrudes from the body portion 31a toward a direction inclined with respect to the extending direction A on the outer side in the radial direction B, and a locking claw 31b2 that protrudes toward the inner side in the radial direction B from a distal end portion which is a tip portion of the arm portion 31b1. The arm portion 31b1 can swing in the radial direction B with a proximal end portion which is a base end portion connected to the body portion 31a, as a fulcrum. An engagement surface 33 that faces the proximal side in the extending direction A is provided on a surface of the arm portion 31b1 on the outer side in the radial direction B. The engagement surface 33 engages with the end surface of the housing body 20 described above on the distal side in the extending direction A. More specifically, the engagement surfaces 33 according to the present embodiment come into contact with and engage with the recessed portions 21c formed in the end surface of the housing body 20 on the distal side in the extending direction A. Since the engagement surfaces 33 engage with the end surface described above of the housing body 20, the movement of the first movable body 31 to the proximal side in the extending direction A with respect to the housing body 20 is restricted.

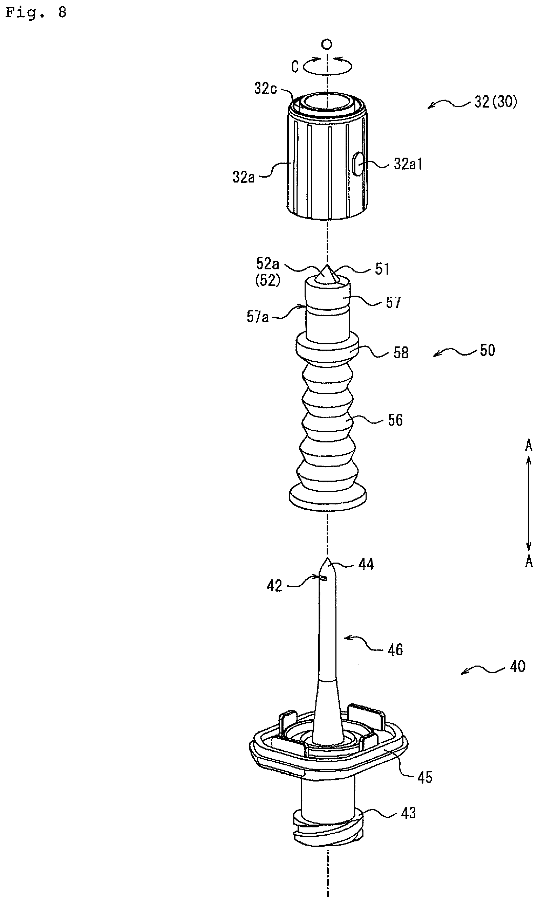

FIG. 8 is an exploded perspective view of the flow passage tubular member 40, the valve body 50, and the second movable body 32 of the male connector 1. As illustrated in FIGS. 4, 5, and 8, the second movable body 32 includes an outer tubular portion 32a having a substantially cylindrical shape, an annular flange portion 32b, and an inner tubular portion 32c.

Protrusion portions 32a1 protruding toward the outer side in the radial direction B are provided on an outer surface of the outer tubular portion 32a. The protrusion portions 32a1 are provided at positions facing each other in the radial direction B. The protrusion portions 32a1 are fitted into the long holes 31a1 of the first movable body 31 described above. The protrusion portions 32a1 are movable in the long holes 31a1 in the extending direction A. Accordingly, the second movable body 32 is movable in the extending direction A with respect to the first movable body 31 in a range where the protrusion portions 32a1 are movable in the long holes 31a1 in the extending direction A.

The flange portion 32b protrudes from an inner surface of the outer tubular portion 32a toward the inner side in the radial direction B. As illustrated in FIGS. 4 and 5, the flange portion 32b is in contact with a surface of a flange portion 58 of the valve body 50 on the distal side. Therefore, when the second movable body 32 is moved to the proximal side in the extending direction A, the flange portion 32b presses the flange portion 58 of the valve body 50 toward the proximal side. Accordingly, by moving the second movable body 32 toward the proximal side in the extending direction A, the valve body 50 can be compressively deformed toward the proximal side in the extending direction A. On the other hand, when the valve body 50 in a state of being compressively deformed toward the proximal side in the extending direction A extends toward the distal side in the extending direction A due to a restoring force, the flange portion 58 of the valve body 50 presses the flange portion 32b of the second movable body 32 toward the distal side. Accordingly, the second movable body 32 can be returned to an original position. Details thereof will be described later.

The inner tubular portion 32c protrudes from an inner edge of the flange portion 32b toward the distal side in the extending direction A. The inner tubular portion 32c is concentrically disposed inside the outer tubular portion 32a in the radial direction B.

An annular protrusion 32c1 protruding toward the inner side in the radial direction B is provided in the inner surface of the inner tubular portion 32c. The annular protrusion 32c1 is fitted into an annular groove 57a (see FIG. 8) on an outer surface of a distal portion 57 of the valve body 50. Accordingly, the distal portion 57 housed in the inner tubular portion 32c is less likely to slip off from the inner tubular portion 32c toward the proximal side in the extending direction A.

As materials of the housing body 20, the first movable body 31, and the second movable body 32 constituting the first housing 10, examples thereof include various resin materials such as polyolefins such as polyethylene, polypropylene, and ethylene-propylene copolymer; ethylenevinyl acetate copolymer (EVA); polyvinyl chloride; polyvinylidene chloride; polystyrene; polyamide; polyimide; polyamideimide; polycarbonate; poly-(4-methylpentene-1); ionomer; acrylic resin; polymethyl methacrylate; acrylonitrile-butadiene-styrene copolymer (ABS resin); acrylonitrile-styrene copolymer (AS resin); butadiene-styrene copolymer; polyesters such as polyethylene terephthalate (PET), polybutylene terephthalate (PBT), and polycyclohexane terephthalate (PCT); polyether; polyether ketone (PEK); polyether ether ketone (PEEK); polyetherimide; polyacetal (POM); polyphenylene oxide; modified polyphenylene oxide; polysulfone; polyether sulfone; polyphenylene sulfide; polyarylate; aromatic polyester (liquid crystal polymer); and polytetrafluoroethylene, polyvinylidene fluoride, and fluorine-based resins. Further, a blend, a polymer alloy, or the like containing one or more of these materials may be used. In addition, various glass materials, ceramic materials, and metal materials may be used.

### [Flow Passage Tubular Member 40]

The flow passage tubular member 40 defines the first flow passage 41 therein. The first flow passage 41 communicates with the outside via the opening 42. The flow passage tubular member 40 is connected to a proximal end of the housing body 20 constituting a proximal end of the first housing 10. Accordingly, a proximal side of the hollow portion 29 of the housing body 20 is sealed by the flow passage tubular member 40. The flow passage tubular member 40 includes, at a proximal end portion, a medical device connecting portion 43 that is connectable to a medical device such as a medical tube. Further, the flow passage tubular member 40 includes the flow passage tubular body 46 that extends into the hollow portion 11 of the first housing 10 and has the opening 42 formed at a distal end portion. The first flow passage 41 communicates from a proximal end of the medical device connecting portion 43 to the opening 42 at the distal end portion of the flow passage tubular body 46. Further, the flow passage tubular member 40 includes, at a distal end portion, a distal portion 44 that is reduced in diameter toward the distal side in the extending direction A. In the present embodiment, the distal portion 44 has a shape formed with two tapered portions having different diameter reduction rates, and may have a different tapered shape such as a conical shape. A distal end of the distal portion 44 may not be sharp and may be formed into a planar shape along the radial direction B.

In addition, the flow passage tubular member 40 includes an annular flange portion 45 protruding toward the outer side in the radial direction B from the medical device connecting portion 43. A proximal end of a bellows tube portion 56 of the valve body 50, which will be described later, is supported by a surface of the flange portion 45 on the distal side.

The flow passage tubular member 40 can be formed of the same material as that of the first housing 10 described above.

### [Valve Body 50]

The valve body 50 covers the flow passage tubular body 46 in the first housing 10. Specifically, the valve body 50 according to the present embodiment includes the bellows tube portion 56 that is elastically deformable in the extending direction A, the distal portion 57 that is continuous with the bellows tube portion 56 so as to close a distal side of a hollow portion of the bellows tube portion 56, and that is housed in the inner tubular portion 32c of the second movable body 32, and the flange portion 58 that protrudes toward the outer side in the radial direction B from the bellows tube portion 56.

A proximal end portion of the bellows tube portion 56 is supported by the flow passage tubular member 40. More specifically, in a state where the bellows tube portion 56 and the distal portion 57 cover the flow passage tubular body 46 of the flow passage tubular member 40, the proximal end of the bellows tube portion 56 is supported while in contact with the surface of the flange portion 45 of the flow passage tubular member 40 on the distal side.

The distal portion 57 is held in the inner tubular portion 32c in the second movable body 32 of the movable body 30. As illustrated in FIG. 8, the annular groove 57a is formed on the outer surface of the diatal portion 57 according to the present embodiment. As described above, the annular protrusion 32c1 (see FIG. 4) provided on the inner surface of the inner tubular portion 32c is fitted into the annular groove 57a on the outer surface of the distal portion 57. Accordingly, the distal portion 57 is less likely to slip off from the inside of the inner tubular portion 32c toward the proximal side in the extending direction A. Further, since the flange portion 58 is in contact with the flange portion 32b of the second movable body 32 described above, the distal portion 57 is not slipped off from the inside of the inner tubular portion 32c toward the distal side in the extending direction A. In this way, the distal portion 57 is maintained in a state of being housed in the inner tubular portion 32c of the second movable body 32.

Further, a slit 51 penetrating from the proximal side to the distal side is formed in the distal portion 57. A conical protrusion 52a protruding toward the distal side is formed at a central portion of a top surface 52 which is an end surface of the distal portion 57 on the distal side. By providing the protrusion 52a on the central portion of the top surface 52, the protrusion 52a is easily brought into close contact with the top surface 72 of the elastic valve body 70 of the female connector 2, which will be described later. Therefore, when the connection of the male connector 1 and the female connector 2 is released, the liquid such as medical fluids hardly adheres to the top surface 52 of the valve body 50 and the top surface 72 of the elastic valve body 70. Details thereof will be described later.

The male connector 1 before connection illustrated in FIGS. 1 to 5 is in the first configuration in which the opening 42 of the flow passage tubular member 40 is closed by the valve body 50. As illustrated in FIG. 4, in the present embodiment, since the distal end portion of the flow passage tubular member 40a including the opening 42 is covered by the valve body 50, the opening 42 is closed.

The hardness of the valve body 50 is preferably a Shore A hardness of 10 or more and 70 or less, and more preferably a Shore A hardness of 20 or more and 50 or less. In a case where the hardness is smaller than a Shore A hardness of 10, when a pressure in the first flow passage 41 is increased, the fluid such as medical fluids may leak to the outside. When the hardness is larger than a Shore A hardness of 70, the contact between the valve body 50 and the elastic valve body 70 of the female connector 2 becomes insufficient, and when the connection with the female connector 2 is released, the fluid such as medical fluids may leak to the outside. In addition, in the case where the hardness of the valve body 50 is the Shore A hardness of 20 or more and 50 or less, it is possible to ensure suitable adhesion with the female connector 2, and when the connection with the female connector 2 is released, it is possible to more reliably restrain the leakage of the fluid such as medical fluids to the outside.

The valve body 50 is formed by metal molding, and is formed to be elastically deformable. Examples of a material for the valve body 50 include various rubber materials such as a natural rubber, an isoprene rubber, a butadiene rubber, a styrene-butadiene rubber, a nitrile rubber, a chloroprene rubber, a butyl rubber, an acrylic rubber, an ethylene-propylene rubber, a hydrin rubber, a urethane rubber, a silicone rubber, and a fluororubber; and various thermoplastic elastomers such as a styrene-based elastomer, a polyolefin-based elastomer, a polyvinyl chloride-based elastomer, a polyurethane-based elastomer, a polyester-based elastomer, a polyamide-based elastomer, a polybutadiene-based elastomer, a transpolyisoprene-based elastomer, a fluororubber-based elastomer, and a chlorinated polyethylene-based elastomer, and one of these may be used alone, or two or more of these may be used in combination. The valve body 50 may also be formed of different materials, or may also be formed of the same material.

### <Female Connector 2>

### [Second Housing 60]

As illustrated in FIGS. 4 and 5, the second housing 60 includes a cap 62, and a holder 63 that supports the cap 62 and that defines the second flow passage 66, and the cap 62 defines the male connector insertion portion 61 into which the male connector 1 is inserted from the outside. The male connector insertion portion 61 is a hollow portion defined by the cap 62 and the holder 63. The second flow passage 66 is a hollow portion defined by the holder 63. The male connector insertion portion 61 is defined closer to the proximal side than the second flow passage 66. The hollow portion defined by the second housing 60 includes the male connector insertion portion 61 and the second flow passage 66.

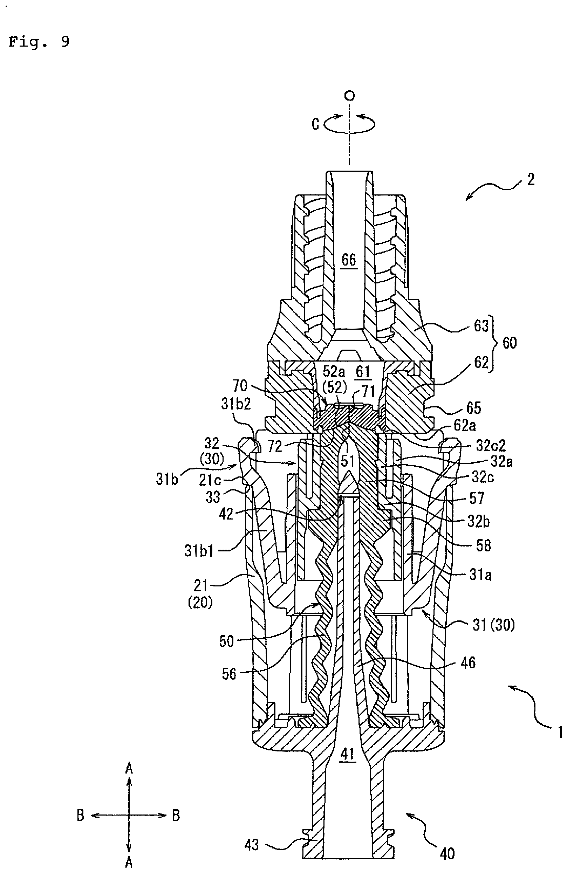

An annular groove 65 serving as a locked portion, into which the locking claws 31b2 of the locking claw portions 31b of the first movable body 31 can be fitted, is formed on an outer peripheral wall of the cap 62. In addition, the locking claws 22b1 of the locking claw portions 22 of the housing body 20 can also be fitted into the annular groove 65. Details of the fitting of the locking claws 31b2 and the locking claws 22b1 into the annular groove 65 will be described later (see FIGS. 9 to 14). The cap 62 is formed by two members that sandwich and fix the elastic valve body 70 from both sides in the extending direction A.

The second housing 60 can be formed of the same material as that of the first housing 10 of the male connector 1 described above.

### [Elastic Valve Body 70]

A slit 71 penetrating from the proximal side to the distal side is formed in the elastic valve body 70. As illustrated in FIGS. 4 and 5, the top surface 72 of the elastic valve body 70 is exposed to the outside. The elastic valve body 70 can be formed of the same material as that of the valve body 50 of the male connector 1 described above. The hardness of the elastic valve body 70 is preferably a Shore A hardness of 20 or more and 60 or less. Further, the hardness of the elastic valve body 70 is preferably larger than the hardness of the valve body 50 of the male connector 1. By setting the hardness of the elastic valve body 70 larger than that of the valve body 50, an amount of deformation of the elastic valve body 70 when the male connector 1 and the female connector 2 are connected can be easily restrained while restraining the leakage of the fluid such as medical fluids to the outside when a pressure in the second flow passage 66 increases. In a case where the amount of deformation of the elastic valve body 70 is large, when the male connector 1 is detached from the female connector 2, a negative pressure may generate in the second flow passage 66, and blood may be sucked from a blood vessel of the patient that is in communication with the second flow passage 66.

### <Connection Operation of Male Connector 1 and Female Connector 2>

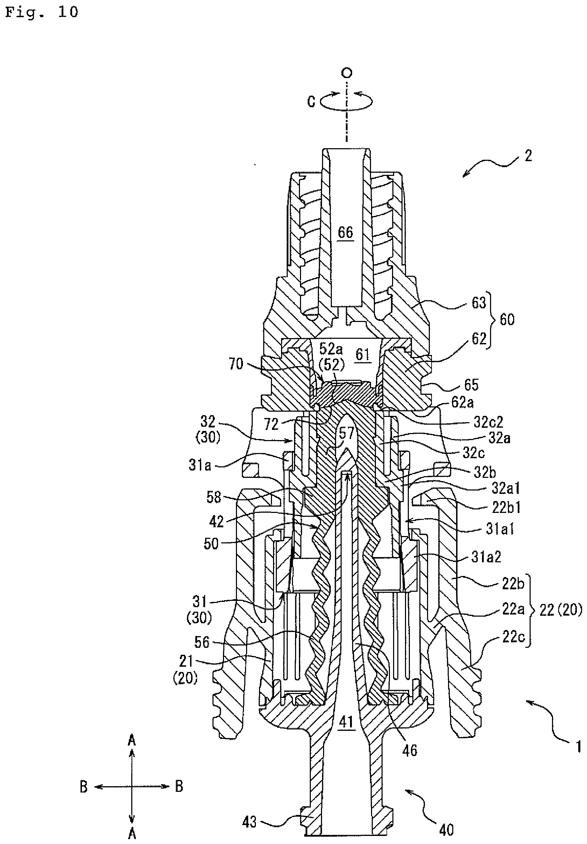

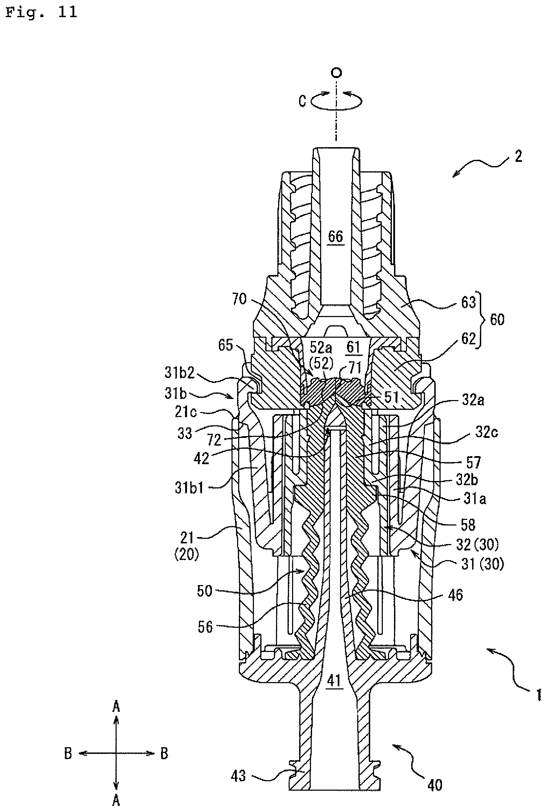

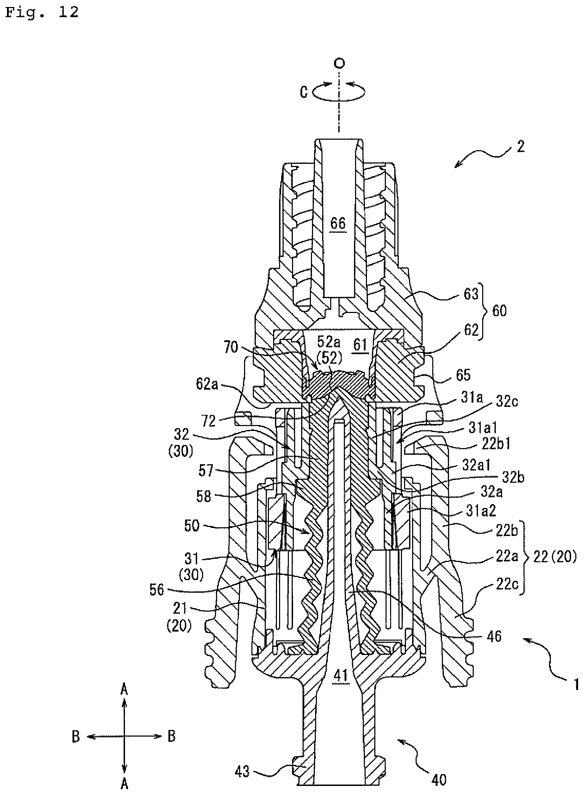

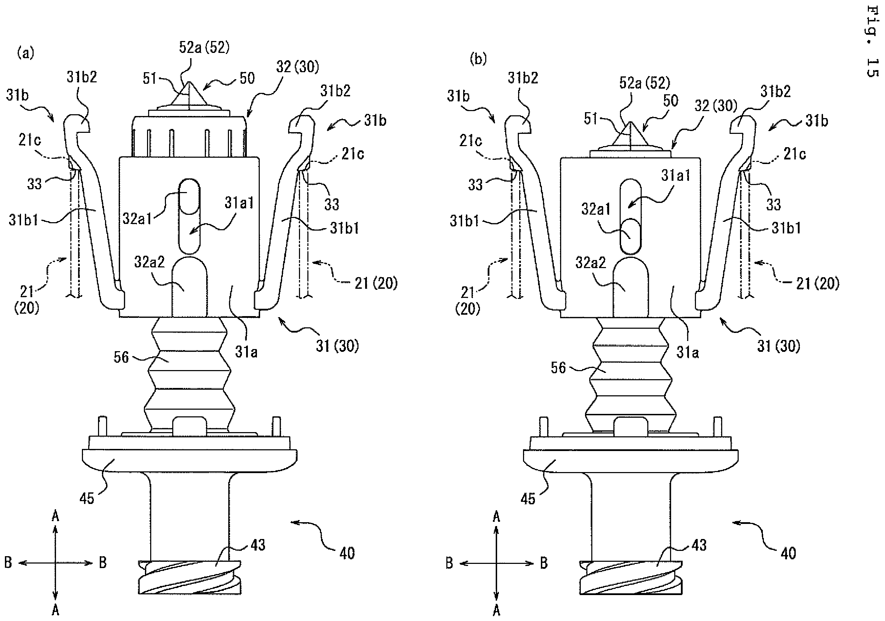

Hereinafter, a connection operation of the male connector 1 and the female connector 2 will be described with reference to FIGS. 4, 5, and 9 to 15. As described above, FIGS. 4 and 5 are cross-sectional views illustrating the state before connection of the male connector 1 and the female connector 2. FIGS. 9, 11, and 13 are cross sections at the same position as in FIG. 4, FIG. 9 illustrates a state of connection in progress, FIG. 11 illustrates a first connection state, and FIG. 13 illustrates a second connection state. Similarly, FIGS. 10, 12, and 14 are cross sections at the same position as in FIG. 5, FIG. 10 illustrates the state of connection in progress, FIG. 12 illustrates the first connection state, and FIG. 14 illustrates the second connection state. FIG. 15 is a view illustrating an operation of the second movable body 32 from the state of connection in progress illustrated in FIGS. 9 and 10 to the first connection state illustrated in FIGS. 11 and 12. Details of the first connection state and the second connection state will be described later.

The male connector 1 and the female connector 2 can be connected by moving to be brought close to each other in the extending direction A from the state before connection illustrated in FIGS. 4 and 5. FIGS. 9 and 10 illustrate a state where the male connector 1 and the female connector 2 are brought close to each other in the extending direction A from the state before connection illustrated in FIGS. 4 and 5, and the top surface 52 of the valve body 50 of the male connector 1 and the top surface 72 of the elastic valve body 70 of the female connector 2 are brought into contact with each other. In this way, when the male connector 1 and the female connector 2 according to the present embodiment are connected, the top surface 52 of the valve body 50 of the male connector 1 and the top surface 72 of the elastic valve body 70 of the female connector 2 first come into contact with each other. When the male connector 1 and the female connector 2 are further brought close to each other in the extending direction A, a top surface 62a of the cap 62 of the female connector 2 comes into contact with a distal end 32c2 of the inner tubular portion 32c of the second movable body 32 of the male connector 1. FIGS. 9 and 10 illustrate a state where the top surface 52 of the valve body 50 of the male connector 1 is in contact with the top surface 72 of the elastic valve body 70 of the female connector 2, and the distal end 32c2 of the second movable body 32 of the male connector 1 is in contact with the top surface 62a of the cap 62 of the female connector 2.

When the male connector 1 and the female connector 2 are further brought close to each other in the extending direction A from the state illustrated in FIGS. 9 and 10, the top surface 62a of the cap 62 of the female connector 2 presses the distal end 32c2 of the inner tubular portion 32c of the second movable body 32 of the male connector 1 toward the proximal side in the extending direction A. Accordingly, the second movable body 32 moves to the proximal side in the extending direction A from a state illustrated in (a) of FIG. 15 to a state illustrated in (b) of FIG. 15. As illustrated in (a) of FIG. 15 and (b) of FIG. 15, in this case, the second movable body 32 relatively moves with respect to the first movable body 31 and the housing body 20. At this time, the first movable body 31 is maintained in a locked state such that the first movable body 31 does not move in the extending direction A with respect to the housing body 20. More specifically, as illustrated in (a) of FIG. 15 and (b) of FIG. 15, a state is maintained where the engagement surfaces 33 of the locking claw portions 31b of the first movable body 31 are in contact with the recessed portions 21c of the end surface of the tubular portion 21 of the housing body 20. Therefore, the first movable body 31 is maintained in the locked state where the first movable body 31 cannot relatively move toward the proximal side in the extending direction A with respect to the housing body 20.

As illustrated in (a) of FIG. 15 and (b) of FIG. 15, the second movable body 32 according to the present embodiment is movable in the extending direction A with respect to the first movable body 31 from a position where the protrusion portions 32a1 are in contact with distal walls of the long holes 31a1 to a position where the protrusion portions 32a1 are in contact with proximal walls of the long holes 31a1. When the second movable body 32 moves from the state illustrated in (a) of FIG. 15 to the state illustrated in (b) of FIG. 15, the valve body 50 is also compressively deformed in the extending direction A. Specifically, the flange portion 32b (see FIG. 4 and the like) of the second movable body 32 presses the flange portion 58 (see FIG. 4 and the like) of the valve body 50 toward the proximal side in the extending direction A. Accordingly, the bellows tube portion 56 of the valve body 50 is elastically deformed and compressed in the extending direction A.

When the second movable body 32 is further pressed toward the proximal side in the extending direction A from the state illustrated in (b) of FIG. 15, the protrusion portions 32a1 of the second movable body 32 presses the proximal walls of the long holes 31a1 toward the proximal side. Accordingly, the engagement surfaces 33 of the locking claw portions 31b of the first movable body 31 move toward the inner side in the radial direction B so as to slide with the recessed portions 21c which are parts of the end surface of the tubular portion 21 of the housing body 20 (see FIG. 11). Accordingly, all of the locking claw portions 31b of the first movable body 31 enter the tubular portion 21 of the housing body 20. That is, the state where the first movable body 31 is locked such that the first movable body 31 does not move toward the proximal side in the extending direction A with respect to the housing body 20 is released. As a result, the first movable body 31 is in a state of being movable toward the proximal side in the extending direction A with respect to the housing body 20.

FIG. 11 illustrates a state where all of the locking claw portions 31b of the first movable body 31 enter the tubular portion 21 of the housing body 20 from the state illustrated in (b) of FIG. 15. As illustrated in FIG. 11, since the locking claw portions 31b of the first movable body 31 move toward the inner side in the radial direction B, the locking claws 31b2 enter the annular groove 65 of the cap 62 of the female connector 2. Accordingly, the first movable body 31 can be locked so as not to move the female connector 2 toward the distal side in the extending direction A. Therefore, it is possible to stably maintain a state where the valve body 50 of the male connector 1 and the elastic valve body 70 of the female connector 2 form a contact region. In the present embodiment, the state where the locking claws 31b2 enter the annular groove 65 of the cap 62 of the female connector 2 is referred to as the "first connection state".

When the male connector 1 and the female connector 2 are further brought close to each other in the extending direction A from the state illustrated in FIGS. 11 and 12, the first movable body 31 and the second movable body 32 further move toward the proximal side in the extending direction A with respect to the housing body 20 while the first movable body 31 is in a state of locking the female connector 2. Accordingly, the bellows tube portion 56 of the valve body 50 is further compressively deformed in the extending direction A. As a result, as illustrated in FIGS. 13 and 14, a distal end of the flow passage tubular body 46 of the flow passage tubular member 40 passes through the contact region of the valve body 50 and the elastic valve body 70, and penetrates the valve body 50 and the elastic valve body 70. Accordingly, the male connector 1 changes the configuration from the first configuration where the valve body 50 closes the opening 42 of the flow passage tubular member 40 (see FIGS. 4, 9, and 11) to the second configuration where the valve body 50 frees the opening 42 of the flow passage tubular member 40 (see FIG. 13). Then, the first flow passage 41 of the male connector 1 and the second flow passage 66 of the female connector 2 are in a state of communicating with each other in a fluid-tight manner.

Then, when the first movable body 31 and the second movable body 32 further move toward the proximal side in the extending direction A with respect to the housing body 20, as illustrated in FIG. 14, the locking claws 22b1 of the locking claw portions 22 of the housing body 20 climb over a part of an outer peripheral surface of the cap 62 of the female connector 2. As a result, the locking claws 22b1 enter the annular groove 65 of the female connector 2. More specifically, since the inclined surfaces 25a of the locking claws 22b1 slide on an outer edge portion of the top surface 62a of the cap 62 of the female connector 2, the locking claws 22b1 are pressed toward the outer side in the radial direction B. Accordingly, the claw body portions 22b move to swing toward the outer side in the radial direction B. As a result, the locking claws 22b1 can move toward the outer side in the radial direction B and can climb over the part of the outer peripheral surface of the cap 62. That is, at the time of the connection operation of the male connector 1 and the female connector 2, the operation portions 22c of the locking claw portions 22 are not required to be operated. In the male connector 1 and the female connector 2 according to the present embodiment, the locking claws 22b1 can be allowed to enter the annular groove 65 of the female connector 2 by only moving the male connector 1 and the female connector 2 in a direction of approaching each other in the extending direction A. Accordingly, while the locking claws 31b2 enter the annular groove 65 (see FIG. 13), as illustrated in FIG. 14, the locking claws 22b1 can also be allowed to enter the annular groove 65. In the present embodiment, the state where the locking claws 31b2 enter the annular groove 65 of the cap 62 of the female connector 2, and the state where the locking claws 22b1 enter the annular groove 65 of the cap 62 of the female connector 2 are referred to as the "second connection state".

By achieving the second connection state described above, the connection of the male connector 1 and the female connector 2 is completed.

### <Detachment Operation of Male Connector 1 and Female Connector 2>

Next, a detachment operation of releasing the connection of the male connector 1 and the female connector 2 in the second connection state illustrated in FIGS. 13 and 14 will be described. At the time of detachment, the operation portions 22c of the locking claw portions 22 of the male connector 1 are pressed and moved toward the inner side in the radial direction B. In this way, the claw body portions 22b positioned opposite to the operation portions 22c with the support portions 22a sandwiched therebetween can be moved to the outer side in the radial direction B with the support portions 22a as the fulcra. Accordingly, the locking claws 22b1 in the annular groove 65 of the cap 62 of the female connector 2 also move toward the outer side in the radial direction B and are detached from the annular groove 65. That is, the second connection state is released.

When the locking claws 22b1 are detached from the annular groove 65, the second movable body 32 is pressed toward the distal side in the extending direction A by an elastic force as a restoring force of the bellows tube portion 56 of the compressively deformed valve body 50. More specifically, the flange portion 58 of the valve body 50 presses the flange portion 32b of the second movable body 32 toward the distal side. Accordingly, the second movable body 32 moves toward the distal side in the extending direction A.

The state where the locking claws 31b2 of the first movable body 31 enter the annular groove 65 of the cap 62 of the female connector 2 is also maintained at this point in time. Therefore, when the second movable body 32 moves toward the distal side in the extending direction A, the first movable body 31 and the female connector 2 also move toward the distal side in the extending direction A by following the second movable body 32.

In this way, since the first movable body 31, the second movable body 32, and the female connector 2 are integrated and move toward the distal side in the extending direction A, the flow passage tubular body 46 of the flow passage tubular member 40 is detached from the valve body 50 and the elastic valve body 70. That is, while the first connection state where the locking claws 31b2 of the first movable body 31 enter the annular groove 65 of the cap 62 of the female connector 2 is maintained, the flow passage tubular body 46 of the flow passage tubular member 40 can be detached from the valve body 50 and the elastic valve body 70. Accordingly, in a state where the contact region of the valve body 50 and the elastic valve body 70 is stably formed, the flow passage tubular body 46 of the flow passage tubular member 40 can be detached from the valve body 50 and the elastic valve body 70 through the contact region. As a result, it is possible to restrain the liquid such as medical fluids from adhering to the top surface 52 of the valve body 50 and the top surface 72 of the elastic valve body 70 after the detachment, in other words, it is possible to restrain the occurrence of "liquid droplets remaining" on the top surface 52 of the valve body 50 and the top surface 72 of the elastic valve body 70.

Then, after the flow passage tubular body 46 of the flow passage tubular member 40 is detached from the valve body 50 and the elastic valve body 70, the engagement surfaces 33 of the locking claw portions 31b of the first movable body 31 reach positions in the extending direction A of the recessed portions 21c which are parts of the end surface of the tubular portion 21 of the housing body 20. At this time, the locking claw portions 31b move toward the outer side in the radial direction B by the elastic force as the restoring force of the arm portions 31b1, and the locking claws 31b2 are detached from the annular groove 65. That is, the first connection state is released. Accordingly, the male connector 1 and the female connector 2 are in a state where the male connector 1 and the female connector 2 can be separated from each other in the extending direction A.

The first movable body 31 and the second movable body 32 return to the state illustrated in FIGS. 4 and 5 by the restoring force of the valve body 50. That is, the first movable body 31 returns to a position where the protrusion portions 31a2 (see FIG. 7 and the like) are in contact with the wall portions 21b1 (see FIG. 6 and the like) of the long groove portions 21b of the housing body 20. Further, the second movable body 32 returns to a position where the protrusion portions 32a1 (see FIGS. 8 and 15 and the like) are in contact with the distal walls of the long holes 31a1 (see FIGS. 7 and 15 and the like) of the first movable body 31. In other words, the second movable body 32 returns to a state where the second movable body 32 protrudes toward the distal side in the extending direction A from the first movable body 31.

As described above, the configuration of the male connector 1 can be changed between the first configuration where the valve body 50 closes the opening 42 of the flow passage tubular member 40 (see FIGS. 4, 5, and 9 to 12) and the second configuration where the valve body 50 frees the opening 42 of the flow passage tubular member 40 (see FIGS. 13 and 14). In the present embodiment, the flow passage tubular body 46 of the flow passage tubular member 40 is entirely covered with the valve body 50, so that the first configuration is achieved. Further, in the present embodiment, the distal portion of the flow passage tubular body 46 of the flow passage tubular member 40 penetrates the valve body 50, and the opening 42 positioned at the distal portion goes outside the valve body 50, so that the second configuration is achieved. A configuration in which the first configuration and the second configuration are achieved is not limited to the configuration according to the present embodiment. Therefore, configurations of the flow passage tubular member 40 and the valve body 50 are not limited to the configurations according to the present embodiment either as long as the first configuration and the second configuration can be achieved.

The movable body 30 of the male connector 1 includes the locking claws 31b2 as first locking portions capable of locking the female connector 2 in the state where the valve body 50 is in contact with the elastic valve body 70 of the female connector 2 in the first configuration. As illustrated in FIG. 11, in the first configuration, the locking claws 31b2 as the first locking portions in the first movable body 31 of the movable body 30 can be fitted into the annular groove 65 of the female connector 2 in the state where the valve body 50 and the elastic valve body 70 are in contact with each other to form the contact region.

Further, the housing body 20 of the male connector 1 includes the locking claws 22b1 as second locking portions capable of locking the female connector 2 at positions different from the positions where the locking claws 31b2 as the first locking portions are locked in the state where the locking claws 31b2 as the first locking portions lock the female connector 2 in the second configuration. As illustrated in FIG. 14, in the second configuration, the locking claws 22b1 as the second locking portions of the housing body 20 can be fitted to other positions of the annular groove 65 while the locking claws 31b2 as the first locking portions are fitted into the annular groove 65 of the female connector 2.

Although the first locking portions according to the present embodiment are implemented by the locking claws 31b2, the configuration of the first locking portions is not particularly limited as long as the movement of the female connector 2 toward the distal side in the extending direction A is restricted so that the female connector 2 is not separated to the distal side in the extending direction A. Further, although the second locking portions according to the present embodiment are implemented by the locking claws 22b1, the configuration of the second locking portions is not particularly limited as long as the movement of the female connector 2 toward the distal side in the extending direction A is restricted so that the female connector 2 is not separated on the distal side in the extending direction A.

Since the configuration of the male connector 1 can be changed between the first configuration and the second configuration, it is possible to restrain the leakage of the fluid such as medical fluids to the outside when the connection with the female connector 2 is released. Further, since the male connector 1 includes the first locking portions and the second locking portions described above, the connection stability with the female connector 2 can be improved.

In addition, in the present embodiment, two locking claws 31b2 of the first movable body 31 as the first locking portions of the movable body 30 are provided at intervals in the circumferential direction C. More specifically, the two locking claws 31b2 according to the present embodiment are disposed at positions facing each other in the radial direction B. In this way, it is preferable that the locking claws 31b2 as the first locking portions are not formed in an endless curved shape such as a tubular shape. In this way, the locking claws 31b2 that easily move toward the radial direction B can be implemented. The number and the positions of the first locking portions are not limited to the number and the positions according to the present embodiment. Only one first locking portion may be provided in a part in the circumferential direction C, or three or more of the first locking portions may be provided at intervals in the circumferential direction C.

In the present embodiment, since the movable body 30 moves in the extending direction A with respect to the housing body 20, the locking claws 31b2 as the first locking portions of the movable body 30 are movable in the radial direction B. More specifically, since the first movable body 31 moves in the extending direction A with respect to the housing body 20, the locking claws 31b2 of the first movable body 31 as the first locking portions of the movable body 30 according to the present embodiment are movable in the radial direction B. As illustrated in FIG. 4 and the like, the engagement surfaces 33 are in contact with and hooked to the recessed portions 21c which are parts of the end surface of the tubular portion 21 of the housing body 20, so that the first movable body 31 according to the present embodiment is locked to the housing body 20, and thus the movement toward the proximal side in the extending direction A is restricted. When the first movable body 31 is pressed toward the proximal side in the extending direction A from this state, the arm portions 31b1 of the locking claw portions 31b of the first movable body 31 are elastically deformed toward the inner side in the radial direction B, and the engagement surfaces 33 described above move toward the inner side in the radial direction B from the recessed portions 21c. Accordingly, as illustrated in FIG. 11 and the like, the locking of the first movable body 31 to the housing body 20 is released. In addition, the locking claws 31b2 as the first locking portions move toward the inner side in the radial direction B. In other words, the locking claws 31b2 as the first locking portions can move toward the inner side in the radial direction B in conjunction with the movement of the first movable body 31 toward the proximal side in the extending direction A (see FIG. 11). On the other hand, when the connection with the female connector 2 is released or the like, the locking claws 31b2 as the first locking portions move toward the outer side in the radial direction B by the restoring force of the arm portions 31b1 in conjunction with the movement of the first movable body 31 toward the distal side in the extending direction A. As a result, the locking claws 31b2 as the first locking portions return to the positions illustrated in FIG. 4 and the like.

The configuration is not limited to the configuration of the locking claws 31b2 according to the present embodiment as long as the first locking portions move in the radial direction B in conjunction with the movement of the movable body 30 in the extending direction A.

Since the movable body 30 of the male connector 1 includes the first locking portions that move in the radial direction B in conjunction with the movement of the movable body 30 in the extending direction A, the locking of the female connector 2 by the first locking portions can be achieved in the connection operation of the male connector 1 and the female connector 2 by the approaching movement in the extending direction A alone.

In the present embodiment, two locking claws 22b1 as the second locking portions of the housing body 20 are provided at intervals in the circumferential direction C. More specifically, the two locking claws 22b1 according to the present embodiment are disposed at positions facing each other in the radial direction B. In this way, it is preferable that the two locking claws 22b1 as the second locking portions are disposed at the positions facing each other. In this way, the female connector 2 can be stably locked. In particular, the locking claws 22b1 according to the present embodiment are released from the locked state with the female connector 2 by pushing the operation portions 22c toward the inner side in the radial direction B. Therefore, when releasing the connection with the female connector 2, a medical staff can easily release the locked state of the locking claws 22b1 from the female connector 2 by an operation of sandwiching the two operation portions 22c disposed at the positions facing each other.

The number and the positions of the second locking portions are not limited to the number and the positions according to the present embodiment. Only one second locking portion may be provided in a part in the circumferential direction C, or three or more of the second locking portions may be provided at intervals in the circumferential direction C.

Further, in the present embodiment, when the state where the locking claws 22bl as the second locking portions of the housing body 20 lock the female connector 2 is released, the movable body 30 moves toward the distal side in the extending direction A with respect to the housing body 20 by the restoring force of the deformed valve body 50. Accordingly, the configuration of the male connector 1 is changed from the second configuration in which the opening 42 is freed to the first configuration in which the opening 42 is closed. That is, according to the male connector 1 of the present embodiment, the configuration can be changed from the second configuration to the first configuration in conjunction with the operation of releasing the locked state of the female connector 2 by the locking claws 22b1 as the second locking portions. Accordingly, when the connection with the female connector 2 is released, the opening 42 of the flow passage tubular member 40 can be more reliably closed by the valve body 50.

In the present embodiment, as described above, the movable body 30 can be returned to an original position by the restoring force of the bellows tube portion 56 of the valve body 50 compressively deformed in the extending direction A, but the disclosure is not limited to this configuration. The male connector 1 may include, for example, a biasing member that biases the valve body 50 or the movable body 30 toward the distal side in the extending direction A. That is, the male connector 1 may return the movable body 30 to the original position by a biasing force of the biasing member.

Further, in the present embodiment, after the configuration changes from the second configuration in which the opening 42 is freed to the first configuration in which the opening 42 is closed, the movable body 30 continuously moves toward the distal side in the extending direction A with respect to the housing body 20 by the restoring force of the valve body 50. Accordingly, the state where the locking claws 31b2 as the first locking portions lock the female connector 2 is released. More specifically, when the connection with the female connector 2 is released, the locking claws 31b2 as the first locking portions according to the present embodiment move toward the outer side in the radial direction B by the restoring force of the arm portions 31b1 in conjunction with the movement of the movable body 30 toward the distal side in the extending direction A (see FIGS. 9 and 11) . Accordingly, the locking claws 31b2 as the first locking portions are detached from the annular groove 65 of the female connector 2, and the state where the female connector 2 is locked by the locking claws 31b2 is released. That is, according to the male connector 1 of the present embodiment, the locked state of the female connector 2 by the locking claws 31b2 as the first locking portions can be released by the restoring force of the valve body 50, and as a result, the female connector 2 can be completely separated from the male connector 1. In other words, in the male connector 1 according to the present embodiment, simply by releasing the locked state of the female connector 2 by the locking claws 22b1 as the second locking portions described above, the configuration can be changed from the second configuration to the first configuration, and the locked state of the female connector 2 by the locking claws 31b2 as the first locking portions can be released. That is, according to the male connector 1 of the present embodiment, when releasing the connection with the female connector 2, a medical staff can easily release the connection with the female connector 2 by the operation of sandwiching the operation portions 22c only. As described above, the male connector 1 according to the present embodiment can be easily connected to the female connector 2 simply by an operation of bringing the male connector 1 close to the female connector 2 in the extending direction A at the time of connecting the female connector 2.

As described above, the movable body 30 according to the present embodiment includes the first movable body 31 and the second movable body 32 that are relatively movable in the extending direction A. Further, as illustrated in (a) of FIG. 15 and (b) of FIG. 15, in the state where the first movable body 31 is locked such that the first movable body 31 does not move in the extending direction A with respect to the housing body 20, the second movable body 32 is movable in the extending direction A with respect to the first movable body 31 and the housing body 20. More specifically, in the state where the first movable body 31 is locked such that the first movable body 31 does not move in the extending direction A with respect to the housing body 20, the second movable body 32 is movable in the extending direction A with respect to the first movable body 31 between a protruding position (see (a) of FIG. 15) and a retracted position (see (b) of FIG. 15). The "protruding position" means a position where the second movable body 32 protrudes together with the valve body 50 from the first movable body 31 toward the distal side in the extending direction A (see (a) of FIG. 15). The "retracted position" means a position where the second movable body 32 is retracted together with the valve body 50 from the protruding position toward the proximal side in the extending direction A (see (b) of FIG. 15).

In this way, since the movable body 30 includes the first movable body 31 and the second movable body 32 that are relatively movable in the extending direction A, the movement of the second movable body 32 between the protruding position and the retracted position described above can be achieved. By setting the second movable body 32 to the protruding position, the top surface 52 of the valve body 50 held by the second movable body 32 can be disposed at a position where the top surface 52 can be easily cleaned from the outside of the male connector 1. In the present embodiment, the top surface 52 of the valve body 50 constitutes the distal end of the whole of the male connector 1.

### <Medical Device Provided with Male Connector 1>

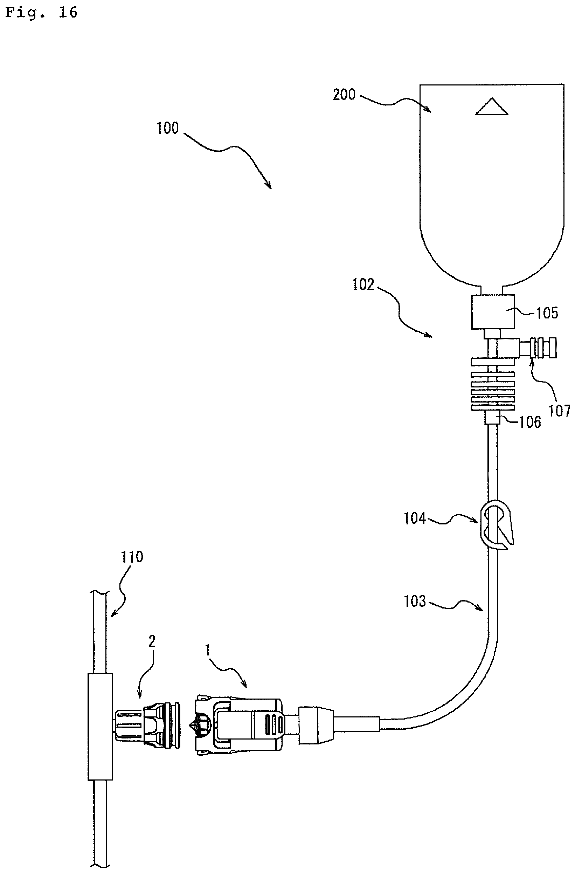

Finally, an infusion tube set 100 as a medical device provided with the male connector 1 described above will be described with reference to FIG. 16. FIG. 16 is a diagram illustrating a state where the infusion tube set 100 provided with the male connector 1 is connected to another infusion tube set 110 provided with the female connector 2 described above. The infusion tube set 100 and the infusion tube set 110 are used for administering an infusion such as medical fluids to a living body. As illustrated in FIG. 16, the infusion tube set 100 includes a connection device 102 connected to a medical fluid container 200, the male connector 1 positioned on a distal side, which is a downstream side than the connection device 102, and a medical tube 103 that connects the connection device 102 and the male connector 1. Further, a clamp 104 that occludes a part of the medical tube 103 may be mounted in the middle of the medical tube 103. The clamp 104 presses the medical tube 103 so as to sandwich the medical tube 103 from the outside, and closes the inside of the medical tube 103 under a pressure.

In the infusion tube set 100 having such a configuration, in a state where the clamp 104 is freed, the liquid such as medical fluids in the medical fluid container 200 flows into the male connector 1 from the connection device 102 through the medical tube 103. Further, when the male connector 1 is connected to the infusion tube set 110 provided with the above-described female connector 2 as a co-infusion port via the female connector 2, for example, the liquid flowing into the male connector 1 passes through the male connector 1 and the female connector 2, flows into the infusion tube set 110, and is supplied to the living body.

The connection device 102 includes a first connection portion 105 that is positioned at a proximal end portion and is connected to the medical fluid container 200, a second connection portion 106 that is positioned at a distal end portion and is connected to the medical tube 103, and a third connection portion 107 that is provided to protrude laterally from an outer wall and is connected with a syringe. A main flow passage that communicates from the first connection portion 105 to the second connection portion 106 and is capable of transporting the liquid in the medical fluid container 200 to the medical tube 103, and a sub flow passage that communicates between the first connection portion 105 and the third connection portion 107 and is capable of transporting the liquid between the medical fluid container 200 and the syringe connected to the third connection portion 107, are defined in the connection device 102.

Therefore, by connecting the syringe in which a medical fluid containing an anti-cancer agent or the like is housed to the third connection portion 107 of the connection device 102, the medical fluid in the syringe can be transported to the medical fluid container 200 via the sub flow passage of the connection tool 102. Then, the medical fluid containing the anti-cancer agent housed in the medical fluid container 200 is supplied to the male connector 1 through the main flow passage of the connection device 102 and the medical tube 103.

As described above, the female connector 2 is connected to the distal side of the male connector 1, and the medical fluid containing the anti-cancer agent is supplied into the infusion tube set 110, whereby the medical fluid containing the anti-cancer agent can be administered to the living body. Further, when the administration of the medical fluid is completed and the connection of the male connector 1 and the female connector 2 is released, the valve body 50 of the male connector 1 is closed, so that the leakage of the medical fluid containing the anti-cancer agent from the distal end of the male connector 1 is restrained.

Although the infusion tube set 100 is described, as an example, as the medical device provided with the male connector 1, the male connector 1 may be used not only for the infusion tube set, but also for other medical devices. For example, the male connector 1 may be used for a syringe at a distal portion of a syringe body. In such a case, for example, the third connection portion 107 of the connection device 102 described above may have the same configuration as that of the female connector 2, and the syringe provided with the male connector 1 may be connected to the third connection portion 107.

The male connector and the medical device provided with the male connector according to the disclosure are not limited to the configurations of the embodiments described above, and can be implemented by various configurations without departing from the disclosure of the claims. Although the valve body 50 according to the embodiments described above has a configuration capable of being compressively deformed in the extending direction A by the bellows tube portion 56, the disclosure is not limited to this configuration. For example, the valve body 50 may have a configuration movable in the extending direction A together with the movable body 30. In addition, the slit 51 of the valve body 50 and the slit 71 of the elastic valve body 70 may not be provided in advance, and may have a configuration capable of communicating with the flow passage tubular member 40 by being punctured.

In the embodiments described above, the locking claws 31b2 as the first locking portions and the locking claws 22b1 as the second locking portions of the male connector 1 are fitted into the same annular groove 65 as the locked portion of the female connector 2 to lock the female connector 2, but the disclosure is not limited to this configuration. That is, the locked portion where the first locking portions of the male connector 1 are engaged and the locked portion where the second locking portions of the male connector 1 are engaged may be separate portions of the female connector 2. Further, the configuration of the locked portion is not limited to the annular groove 65 according to the present embodiment, and can be appropriately changed according to the configurations of the first locking portion and the second locking portion.

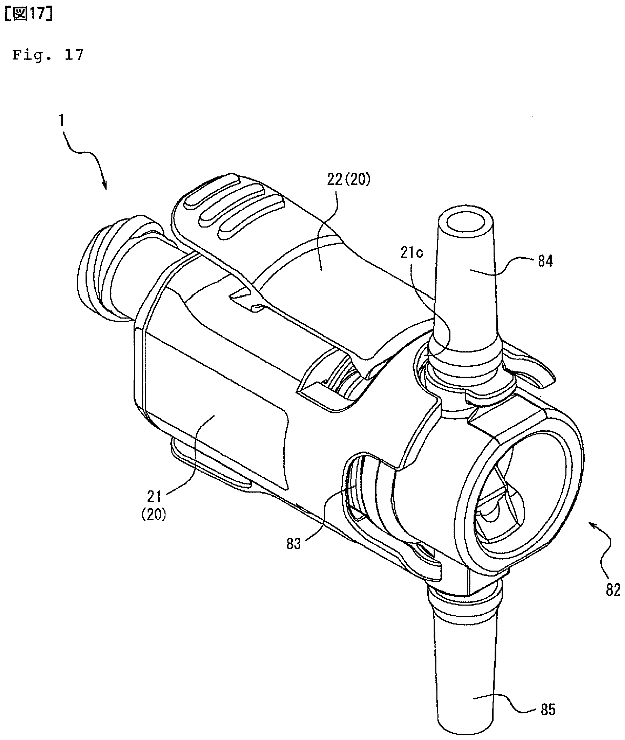

In the embodiments described above, a so-called "I-type connector" provided with the straight type second flow passage 66 is described and exemplified as the female connector 2 connectable to the male connector 1, but the disclosure is not limited to this configuration. The female connector 2 connectable to the male connector 1 may be, for example, a T-shaped female connector including an upstream port, a downstream port, and a co-infusion port connectable to the male connector 1. Further, the male connector 1 according to the embodiments described above is connectable not only to the straight type female connector 2 illustrated in FIG. 1 and the like but also to a T-shaped female connector 82 as illustrated in FIG. 17. A co-infusion port 83 of the T-shaped female connector 82 has the same configuration as that of a part connectable to the male connector 1 of the female connector 2 described in the embodiments described above. In a state where the male connector 1 is connected to the co-infusion port 83, an upstream port 84 and a downstream port 85 in the T-shaped female connector 82 extend to the outside through the positions of the remaining two recessed portions 21c, which are not engaged with the locking claw portions 31b (see FIG. 1 and the like) in the state before connection (see FIG. 4), among the four recessed portions 21c formed in the end surface of the tubular portion 21 of the housing body 20. In the male connector 1 according to the embodiments described above, the two recessed portions 21c engaged with the locking claw portions 31b (see FIG. 1 and the like) in the state before connection (see FIG. 4) are disposed at positions facing each other in the radial direction B. Further, in the male connector 1 according to the embodiments described above, the two recessed portions 21c that receive the upstream port 84 and the downstream port 85 of the female connector 82 are also disposed at positions facing each other in the radial direction B.

### Industrial Applicability

The disclosure relates to a male connector and a medical device.

### Reference Signs List

1: male connector
2: female connector
10: first housing
11: hollow portion
20: housing body
21: tubular portion
21a: opening
21b: long groove portion
21b1: wall portion
21c: recessed portion
22: locking claw portion
22a: support portion
22b: claw body portion
22b1: locking claw (an example of a second locking portion)
22c: operation portion
25a: inclined surface
25b: hook surface
29: hollow portion
30: movable body
31: first movable body
31a: body portion
31a1: long hole
31a2: protrusion portion
31b: locking claw portion
31b1: arm portion
31b2: locking claw (an example of a first locking portion)
32: second movable body
32a: outer tubular portion
32a1: protrusion portion
32b: flange portion
32c: inner tubular portion
32c2: annular protrusion
32c2: distal end
33: engagement surface
39: hollow portion
40: flow passage tubular member
41: first flow passage
42: opening
43: medical device connecting portion
44: distal portion
45: flange portion
46: flow passage tubular body
50: valve body
51: slit
52: top surface
52a: protrusion
56: bellows tube portion
57: distal portion
57a: annular groove
58: flange portion
60: second housing
61: male connector insertion portion
62: cap
62a: top surface
63: holder
65: annular groove
66: second flow passage
70: elastic valve body
71: slit
72: top surface
82: female connector
83: co-infusion port
84: upstream port
85: downstream port
100: infusion tube set (an example of a medical device provided with a male connector)
110: infusion tube set
102: connection device
103: medical tube
104: clamp
105: first connection portion
106: second connection portion
107: third connection portion
A: extending direction of flow passage tubular member
B: radial direction of flow passage tubular member
C: circumferential direction of flow passage tubular member
O: central axis of flow passage tubular member

## Claims

1. A male connector connectable to a female connector provided with an elastic valve body, comprising:
a housing configured to define a hollow portion;
a flow passage tubular member extending in the hollow portion and having an opening formed at one end side in an extending direction; and
a valve body positioned in the hollow portion and configured to close the opening of the flow passage tubular member, wherein
the housing includes
a housing body, and
a movable body configured to move in the extending direction with respect to the housing body to deform or move the valve body such that a configuration is changed between a first configuration in which the opening of the flow passage tubular member is closed by the valve body, and a second configuration in which the opening of the flow passage tubular member is freed from the valve body,
the movable body includes a first locking portion configured to lock the female connector in a state where the valve body is in contact with the elastic valve body of the female connector in the first configuration, and
the housing body includes a second locking portion configured to lock the female connector in a state where the first locking portion locks the female connector in the second configuration.

2. The male connector according to claim 1, wherein
the movable body is provided with only one first locking portion or a plurality of the first locking portions at intervals in a circumferential direction of the flow passage tubular member.

3. The male connector according to claim 1 or 2, wherein
when the movable body moves in the extending direction with respect to the housing body, the first locking portion of the movable body is movable in a radial direction of the flow passage tubular member.

4. The male connector according to any one of claims 1 to 3, wherein
the housing body is provided with only one second locking portion or a plurality of the second locking portions at intervals in a circumferential direction of the flow passage tubular member.

5. The male connector according to any one of claims 1 to 4, wherein
when a state where the second locking portion of the housing body locks the female connector is released, the movable body moves in the extending direction with respect to the housing body due to a restoring force of the deformed valve body or a biasing force of a biasing member that biases the valve body or the movable body toward the one end side in the extending direction, and the configuration is changed from the second configuration to the first configuration.

6. The male connector according to claim 5, wherein,
after the configuration is changed from the second configuration to the first configuration, the movable body continuously moves in the extending direction with respect to the housing body due to the restoring force or the biasing force, so that the state where the first locking portion locks the female connector is released.

7. The male connector according to any one of claims 1 to 6, wherein
the movable body includes a first movable body and a second movable body that are relatively movable in the extending direction, and
in a state where the first movable body is locked such that the first movable body does not move in the extending direction with respect to the housing body, the second movable body is movable in the extending direction with respect to the first movable body and the housing body.

8. A medical device comprising:
the male connector according to any one of claims 1 to 7.
